# EUROPEAN PATENT APPLICATION

(11) **EP 1 600 877 A2**
(43) Date of publication of application: **30.11.2005**
(21) Application number: 05075772.3
(22) Date of filing: 04.04.2005
(51) Int. Cl.: G06F 19/00

(54) **System for processing patient medical data for clinical trials and analysis**

(30) Priority: 28.05.2004 US 575289 P; 17.11.2004 US 991175
(71) Applicant: Siemens Medical Solutions Health Services Corporation, Malvern, PA 19355 (US)
(72) Inventor: Schmidt, Markus, 90419 Nürnberg (DE); Zahlmann, Gudrun Dr., 92318 Neumarkt (DE)
(74) Representative: Condon, Neil

(57) **Abstract**

A system processes medical information of multiple patients to provide data for use in supporting clinical decision making. The system includes an interface for receiving, from a remote source, data representing patient non-specific medical information excluding patient specific information. The patient non-specific medical information comprises medical information of multiple individual patients treated at one or more healthcare organization sites. A data processor automatically, parses the received data, identifies, within the parsed received data, site specific information associated with a particular healthcare provider organization site and facilitates identification of the particular healthcare provider organization site. The data processor removes the identified site specific information from the data representing patient non-specific medical information to provide both patient and site non-specific, patient medical information representative data and stores both the patient and site non-specific, patient medical information representative data in a repository.

## Description

This is a non-provisional application of provisional application serial No. 60/575,289 by M. Schmidt et al. filed May 28, 2004.

### Field of the Invention

This invention concerns a system and user interface supporting data management for clinical trials involving trial sponsors and other participants.

### Background Information

Patients are recruited for healthcare clinical trials in various ways. Patients may be approached by marketing methods (mass media advertising, direct-to-consumer marketing, etc.) or patient data for existing patients in existing databases may be searched to identify suitable trial candidates. A service provider organization supporting performance of clinical trials, needs to establish a database with a large number of patient data sets in order to offer a service for identifying and recruiting candidates for a clinical trial. These data sets are supplied by healthcare providers that maintain repositories containing patient clinical parameters that are needed for a query based search to identify suitable clinical trial candidates.

There are a number of difficulties involved in acquiring patient data sets of candidates suitable for a particular clinical trial. Regulatory requirements forbid the use of patient data sets that contain patient specific identification information. Further, in order to acquire sufficient candidates for a reasonably sized trial, datasets typically need to be acquired from multiple different healthcare providers at multiple different locations and employing different patient record formats, different database management applications as well as different communication protocols. The different healthcare provider systems involved also typically use incompatible technology provided by different vendors, employing different data formats as well as different user interfaces and quality control processes, for example.

Existing systems employ largely manual processes, lack interface technology and fail to provide comprehensive integrated clinical trial data acquisition and processing support. A system according to invention principles addresses the identified deficiencies and related problems.

### Summary of the Invention

A system processes clinical data provided by healthcare providers to identify both patient and medical sites that are suitable candidates for a clinical trial and renders patient medical information anonymous and enables patient data re-identification. A system processes medical information of multiple patients to provide data for use in supporting clinical decision making. The system includes an interface for receiving, from a remote source, data representing patient non-specific medical information excluding patient specific information. The patient non-specific medical information comprises medical information of multiple individual patients treated at one or more healthcare organization sites. A data processor automatically, parses the received data, identifies, within the parsed received data, site specific information associated with a particular healthcare provider organization site and facilitates identification of the particular healthcare provider organization site. The data processor removes the identified site specific information from the data representing patient non-specific medical information to provide both patient and site non-specific, patient medical information representative data and stores both the patient and site non-specific, patient medical information representative data in a repository.

### Brief Description of the Drawing

Figure 1 shows a data exchange system connecting a healthcare provider (HP) and a service provider (SP) managing data for a clinical trial, according to invention principles.
Figure 2 shows a second data exchange system connecting a healthcare provider (HP) and a service provider (SP) involving encryption of site and patient identification information in managing data for a clinical trial, according to invention principles.
Figures 3A, 3B and 3C shows flowcharts of processes used for managing data for a clinical trial, according to invention principles.
Figure 4 shows a database structure used in the system of Figure 1 for managing data for a clinical trial, according to invention principles.
Figure 5 shows a database structure used in the system of Figure 2 for managing data for a clinical trial, according to invention principles.
Figure 6 shows a flowchart of a process used by a Healthcare Provider (HP) for managing data for a clinical trial, according to invention principles.
Figure 7 shows a flowchart of a process used by a Service Provider (HP) for managing data for a clinical trial, according to invention principles.
Figure 8 shows a flowchart of a process used by a service provider (SP) for providing data for a clinical trial in response to an information request, according to invention principles.

### Detailed Description of Invention

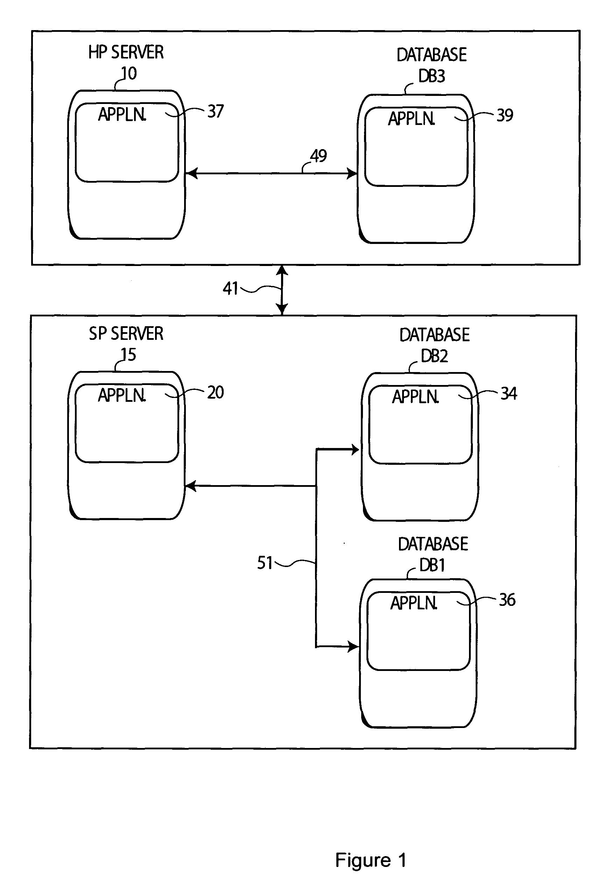

Figure 1 shows a data exchange system connecting a healthcare provider (HP) and a service provider (SP) managing data for a clinical trial. A clinical trial is operated by a Sponsor. A Sponsor is an organization or individual operating a clinical trial for the purpose of acquiring data advancing medical knowledge concerning, causes of medical conditions or treatments and therapies for medical conditions. The system renders patient specific information anonymous for applications in the health care field and ensures compliance with regulatory guidelines. The system involves hierarchical de-identification and re-identification of clinical data by, for example, extraction and subsequent re-insertion of patient and trial site identifiers in clinical data records in a clinical trial. A system searches databases of information derived from data provided by healthcare providers to identify both patient and medical sites that are suitable candidates for a clinical trial. A service provider (SP) provides a business service for rendering patient medical information anonymous and enabling clinical data re-association with a patient and trial site. Although the invention is described in the context of clinical trials, this is exemplary only. The invention covers any applications that relate to searching patient information in the field of health care.

An executable application as used herein comprises code or machine readable instruction for implementing predetermined functions including those of an operating system, healthcare information system or other information processing system, for example, in response user command or input. An executable procedure is a segment of code (machine readable instruction), sub-routine, or other distinct section of code or portion of an executable application for performing one or more particular processes and may include performing operations on received input parameters (or in response to received input parameters) and provide resulting output parameters. A processor as used herein is a device and/or set of machine-readable instructions for performing tasks. A processor comprises any one or combination of, hardware, firmware, and/or software. A processor acts upon information by manipulating, analyzing, modifying, converting or transmitting information for use by an executable procedure or an information device, and/or by routing the information to an output device. A processor may use or comprise the capabilities of a controller or microprocessor, for example. A display processor or generator is a known element comprising electronic circuitry or software or a combination of both for generating display images or portions thereof. A user interface comprises one or more display images enabling user interaction with a processor or other device.

In the Figure 1 system, patient clinical data maintained in a repository (not shown to preserve drawing clarity) is processed by executable application 37 operating on healthcare provider server 10. Specifically, application 37 processes patient clinical data in the form of patient medical information records retrieved from the repository by removing patient identification information from the retrieved clinical data to provide patient non-specific medical information comprising medical information records of multiple individual patients treated at a healthcare provider site. Application 37 allocates, individual new patient identifiers, (e.g., codes of alphanumeric character) different to the removed patient identification information, to corresponding individual patient non-specific medical information records and corresponding removed patient identification information records. The allocated patient identifiers enable application 37 to subsequently re-associate corresponding individual patient non-specific medical information records and corresponding removed patient identification records. The removed patient identification information is communicated together with corresponding allocated individual new patient identifiers to database management application 39 via communication link 49. Application 39 stores the removed patient identification information together with corresponding allocated individual new patient identifiers in database DB3. The removed patient identification information comprises patient specific information such as a text string associated with a patient name, a patient identifier, a medical record number, a patient address, patient contact information, patient medical insurance information and data associated with a patient healthcare provider organization or physician.

At a Service Provider site, executable application 20 operating on server 15 acquires, via communication link 41, the patient non-specific medical information records together with corresponding allocated new patient identifiers provided by application 37. Application 20 removes site identification information from the patient non-specific medical information to provide patient and site non-specific medical information. Application 20 communicates the removed site identification, together with the received corresponding allocated new patient identifiers, to database management application 34. Application 20 communicates the resultant patient and site non-specific medical information, together with the received corresponding allocated new patient identifiers, to database management application 36 via network 51. Database management application 34 stores the removed site identification information and corresponding allocated new patient identifiers in database DB2. Database management application 36 stores the patient and site non-specific medical information and corresponding allocated new patient identifiers in database DB 1.

The same new patient identifiers created and allocated by application 37 are associated with records stored in databases DB3, DB2 and DB1 These allocated patient identifiers enable application 20 to subsequently re-associate corresponding individual site and patient non-specific medical information records and corresponding removed site identification records. Similarly, these allocated patient identifiers enable application 37 to subsequently re-associate corresponding individual patient non-specific medical information records and corresponding removed patient identification records. As a result, application 20 at a service provider location is able to re-associate healthcare provider organizations and their site locations (using information in database DB2) with clinical data in DB1. Application 37 at a healthcare provider location is able to re-associate this clinical data (and re-associated site location information) with individual patients using patient identification information in database DB3.

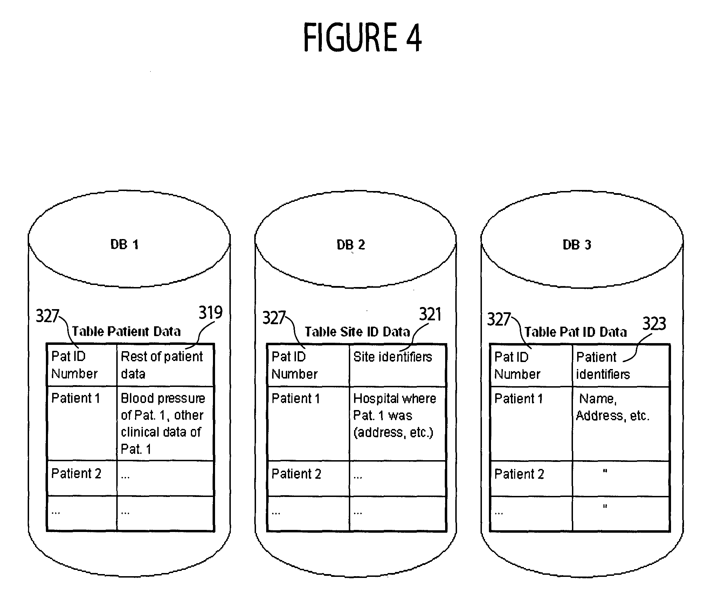

Figure 4 illustrates a structure of databases DB 1, DB2 and DB3 used in the system of Figure 1 for managing data for a clinical trial. Database DB3 associates removed patient identification information in individual records in column 323 with new patient identifiers allocated by application 37 (Figure 1) in column 327 (Figure 4). Database DB2 associates removed site identification information in individual records in column 321 with new patient identifiers allocated by application 37 in column 327. Similarly, database DB1 associates site and patient non-specific medical information records in column 319 with new patient identifiers allocated by application 37 in column 327.

In another embodiment, application 20 allocates a second patient identifier to individual removed site identification records that are stored in database DB2 and to patient and site non-specific medical information that are stored in database DB 1. In this embodiment, application 20 at a service provider location uses the allocated second patient identifiers to re-associate healthcare provider organizations and their site locations (using information in database DB1) with clinical data in DB2. Application 37 at a healthcare provider uses the new patient identifiers allocated by application 37 to re-associate this clinical data (and re-associated site location information) with individual patients using patient identification information in database DB3. In other embodiments, applications 20 and 37 may be located at a single site of a healthcare provider or service provider, for example or may be a single application or a distributed array of applications at different locations.

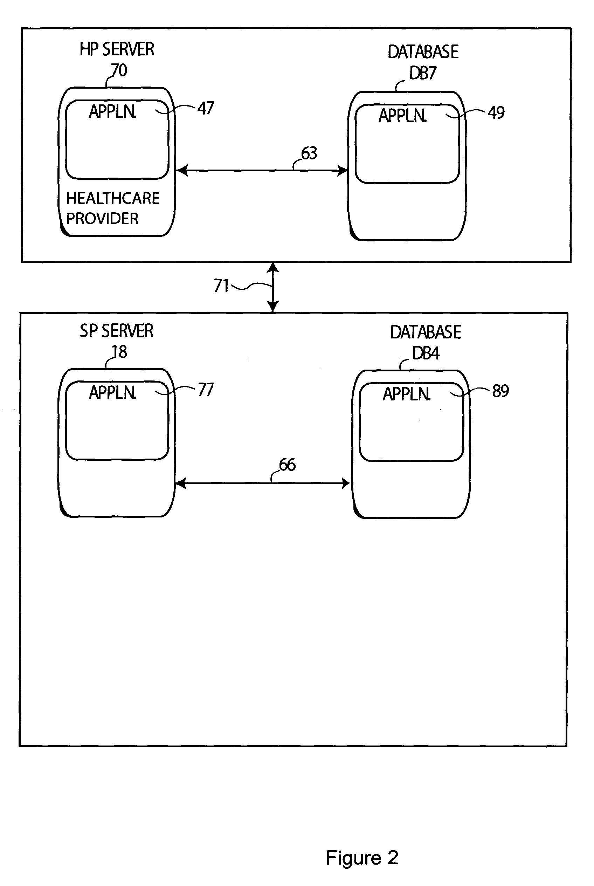

Figure 2 shows a second data exchange system connecting a healthcare provider (HP) and a service provider (SP) involving encryption of patient and site identification information in managing data for a clinical trial. In the Figure 2 system patient clinical data maintained in a repository (not shown to preserve drawing clarity) is processed by executable application 47 operating on healthcare provider server 70. Specifically, application 47 processes patient clinical data in the form of patient medical information records retrieved from the repository by encrypting patient identification information within the patient medical records with a first encryption key (key A). Encryption key A is known by the healthcare provider and is unknown to the service provider. Application 47 communicates, the encrypted patient identification information and patient medical records, on link 63 for storage in database DB7 via database management application 49.

At a Service Provider site, executable application 77 operating on server 18 acquires, via communication link 71, the patient medical information records including the corresponding encrypted patient identifiers provided by application 47. Application 77 encrypts site identification information within the patient medical information records with a second encryption key (key B) known to the service provider and different from the first encryption key known to the healthcare provider. Application 47 communicates the patient medical information records including the encrypted site identification and patient identification information to database management application 89 via network link 66. Database management application 89 stores the patient medical information records, including the encrypted site identification and patient identification information, in database DB4. In other embodiments, applications 70 and 77 may be located at a single site of a healthcare provider or service provider, for example or may be a single application or a distributed array of applications at different locations. Also database DB7 and DB4 may be a single database.

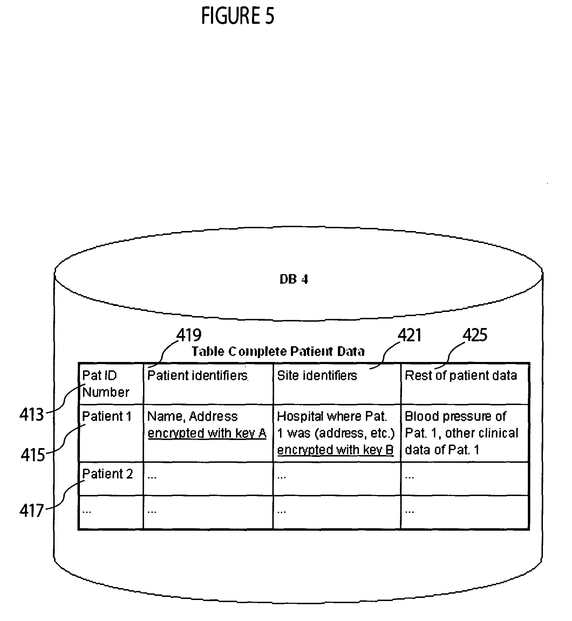

Figure 5 shows a database structure used in the system of Figure 2 for managing data including encrypted site and patient identification information for a clinical trial. Figure 5 illustrates a structure of database DB4 used in the system of Figure 2. Database DB4 associates encrypted patient identification information in individual patient records in column 419 with corresponding encrypted healthcare provider site location identification information in individual patient records in column 421 and with corresponding patient medical information in individual patient records in column 425. Database DB4 similarly associates newly allocated non-encrypted patient identifiers in column 413 (e.g., of patient records of rows 415 and 417) with the records of columns 419, 421 and 425, though in other embodiments such newly allocated patient identifiers may not be used. In such a case, patient identifiers in column 419 following their decryption are used for identifying patient records. Database DB4 associates the various data fields of patient record information of columns 413, 419, 421 and 425 of individual patient record rows (in rows 415 and 417, for example). A complete patient data set is stored in database DB4 that resides at a Service Provider site location However patient identification information data fields in column 419 are encrypted with a key A that is accessible by the healthcare provider (and not the Service Provider) so that only the healthcare provider can re-associate the patient medical information records with their corresponding true patient identification information. The site identification information fields are encrypted with key B that is only accessible by a Service Provider. This means that clinical trial Sponsors are able to access the clinical information in database DB4 without being able to identify particular patients or healthcare provider site locations enabling blind or double-blind trials to be performed.

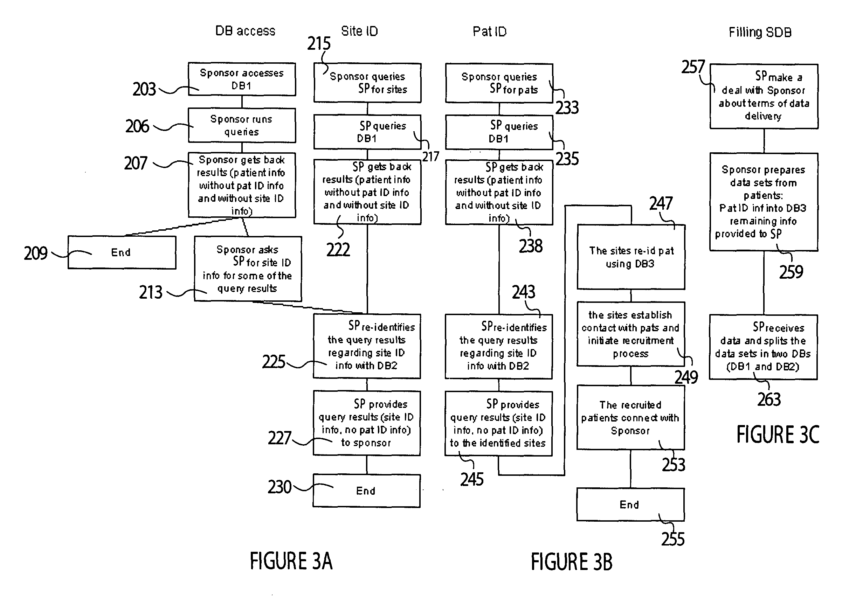

Figures 3A-3C show flowcharts of processes provided by a Service Provider to a clinical trial Sponsor and employing the database structures of Figures 4 and 5 and systems of Figures 1 and 2 for managing data for a clinical trial. In Figure 3A, a Sponsor in activity 203 accesses patient and site non-specific medical data in database DB1 via database management application 36 (Figures 1 and 4) in order to search in step 206 for particular clinical data based on search criteria comprising a search query entered by a user. In response to the query the Sponsor acquires patient and site non-specific medical data in step 207 from database DB1. The Sponsor may end this process at step 209 or may request healthcare provider site location identification information in step 213 indicating the site locations associated with the clinical data acquired in step 207 by sending a query message to a Service Provider in step 213. In this case the process continues with step 225.

In step 215 a Sponsor sends a query message to Service Provider application 20 of server 15 (Figure 1) requesting healthcare provider site location identification information. Service Provider application 20 sends a query message to database DB1 management application 36 in step 217. The Service Provider in step 222 acquires patient and site non-specific medical data in response to the query submitted in step 217. In step 225 Service Provider application 20 retrieves healthcare provider site location identification data for the acquired patient and site non-specific medical data records (clinical data records for multiple different patients), from database DB2 using database management application 34. Thereby, Service Provider application 20 re-associates the acquired patient and site non-specific medical data records with corresponding healthcare provider site location identification information to provide patient non-specific medical data records. Application 20 communicates the patient non-specific medical data records to the requesting Sponsor in step 227 and this process ends at step 230.

In the patient recruitment process of Figure 3B, a Sponsor in step 233, sends a query message, to Service Provider application 20 of server 15 (Figure 1), requesting patient identification information. Service Provider application 20 sends a query message to database DB1 management application 36 in step 235. The Service Provider in step 238 acquires patient and site non-specific medical data in response to the query submitted in step 235. In step 243 Service Provider application 20 retrieves healthcare provider site location identification data for the acquired patient and site non-specific medical data records (clinical data records for multiple different patients), from database DB2 using database management application 34. Thereby, Service Provider application 20 re-associates the acquired patient and site non-specific medical data records with corresponding healthcare provider site location identification information to provide patient non-specific medical data records. Application 20 in step 245 communicates the patient non-specific medical data records to the corresponding healthcare provider site locations identified in step 243.

In step 247, application 37 of an individual healthcare provider organization re-associates the patient non-specific medical data records with individual patients using patient identification information in database DB3 accessed via database management application 39. Individual healthcare provider organizations establish contact with identified patients in step 249 in order to recruit the identified patients for use in a clinical trial. The recruited patients are placed in contact with the Sponsor in step 253 and the process terminates at step 255.

In the Figure 3C process, the Sponsor organization in step 257 makes an agreement with the Service Provider concerning terms of data delivery. The Sponsor prepares data sets comprising medical data records of patients in step 259. The patient identification information is removed and stored in database DB3. The remaining patient non-specific medical data record information is provided to the Service Provider. In step 263 the Service Provider splits the received medical data record information by storing the site identification information in database DB2 and the patient and site non-specific medical record in DB 1.

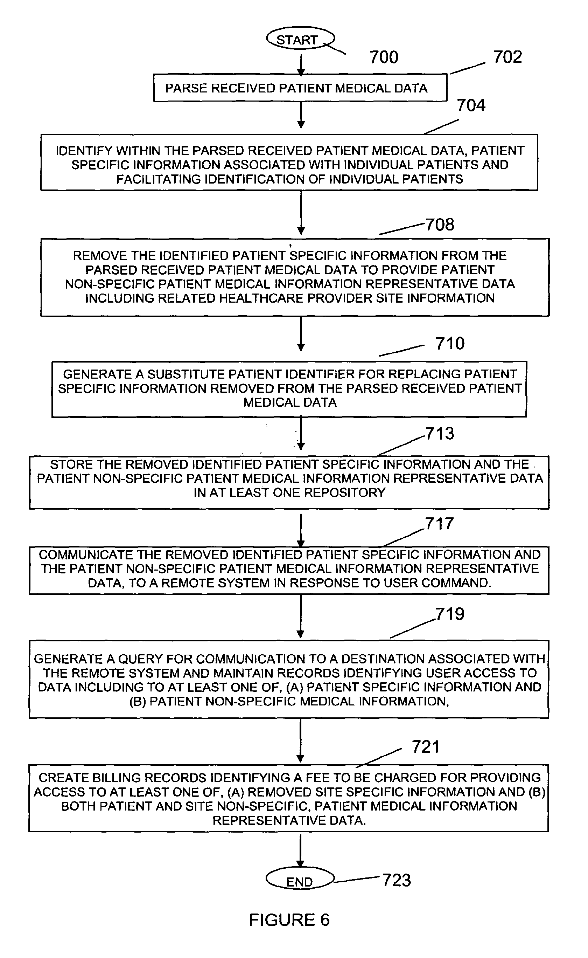

Figure 6 shows a flowchart of a process used by a Healthcare Provider (HP) for managing data for a clinical trial. In step 702 following the start at step 700 application 37 (Figure 6) parses received patient medical data. Application 37 in step 704 identifies, within the parsed received patient medical data, patient specific information that is associated with individual patients and that facilitates identification of individual patients. The patient specific information associated with individual patients comprises at least one of, a text string associated with a patient name, a patient identifier, patient address and patient contact information and is used for identifying particular individual patients associated with corresponding medical data.

In step 708, application 37 removes the identified patient specific information from the parsed received patient medical data to provide patient non-specific patient medical information representative data including related healthcare provider site location information. In another embodiment the step of removing the identified patient specific information comprises encrypting the identified patient specific information. In this embodiment the identified patient specific information comprises encrypted identified patient specific information. Application 37 in step 710 generates a substitute patient identifier for replacing patient specific information removed from the parsed received patient medical data. The substitute patient identifier is linked with a particular patient by stored linking data and is otherwise unassociated with the particular patient. In step 713 application 37 stores the removed identified patient specific information in database DB3 and the patient non-specific patient medical information representative data in database DB3 or another database. Application 37 in step 717 communicates the removed identified patient specific information and the patient non-specific patient medical information representative data, to a remote system in response to user command.

Application 37 in step 719 generates data representing a message for querying the remote system for information required by a trial Sponsor or trial investigator, for example. Application 37 also maintains records identifying user access to data including patient specific information and patient non-specific patient medical information representative data, for example. The records include one or more of, identification information associated with a user attempting to access the data, identification information of data accessed and identification information of a device source of a request to access the data. In step 721 application 37 creates billing records identifying a fee to be charged for providing access to removed patient specific information or patient non-specific patient medical information representative data, for example. The process of Figure 6 terminates at step 723.

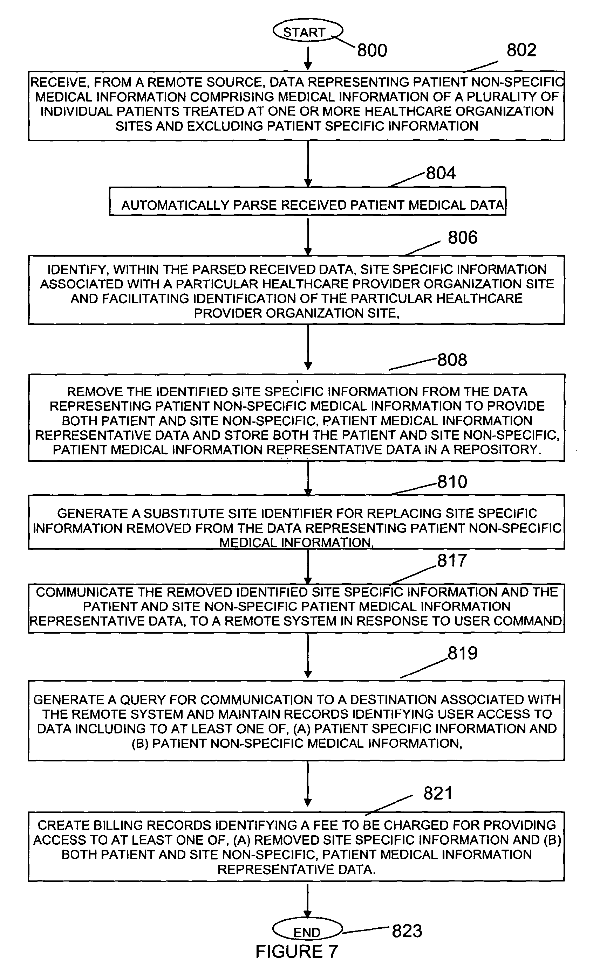

Figure 7 shows a flowchart of a process used by a Service Provider (SP) for managing data for a clinical trial. In step 802 following the start at step 800, application 20 receives, from a remote source, data representing patient non-specific medical information excluding patient specific information. The patient non-specific medical information comprises medical information of multiple individual patients treated at one or more healthcare organization sites. The excluded patient specific information is associated with individual patients and comprises one or more of, (a) a text string associated with a patient name, (b) a patient identifier, (c) patient address, (d) patient contact information, (e) patient medical insurance information, (f) a medical record number and (g) data associated with a patient healthcare provider organization or physician. In step 804 application 20 automatically parses the received data and in step 806 identifies, within the parsed received data, site specific information associated with a particular healthcare provider organization site and facilitating identification of the particular healthcare provider organization site.

Application 20 in step 808 removes the identified site specific information from the data representing patient non-specific medical information to provide both patient and site non-specific, patient medical information representative data. Application 20 stores the patient and site non-specific, patient medical information representative data in database DB1. The removed identified site specific information is stored in database DB2. In an alternative embodiment application 20 encrypts the identified site specific information with a secure encryption key and stores it in database DB4 (instead of removing it). In step 810 application 20 generates a substitute site identifier for replacing site specific information removed from the data representing patient non-specific medical information. The substitute site identifier is linked with a particular healthcare provider organization site by stored linking data and is otherwise unassociated with the particular healthcare provider organization site. In step 817, application 20 retrieves site information associated with selected patient and site non-specific, medical information records from database DB2 and initiates communication of the retrieved site information to a remote system in response to user command.

In step 819, application 20 generates a query requesting access to patient specific information associated with corresponding patient non-specific medical information and communicates the query to a destination associated with the remote system. Application 20 acquires the requested patient specific information in response to the request for the patient specific information. Application 20 maintains records identifying user access to data including to at least one of, (a) patient specific information and (b) patient non-specific medical information. The records include one or more of, (i) identification information associated with a user attempting to access the data, (ii) identification information of data accessed and (iii) identification information of a device source of a request to access the data. Application 20 in step 821 creates billing records identifying a fee to be charged for providing access to at least one of, (a) removed site specific information and (b) both patient and site non-specific, patient medical information representative data. The process of Figure 7 terminates at step 823.

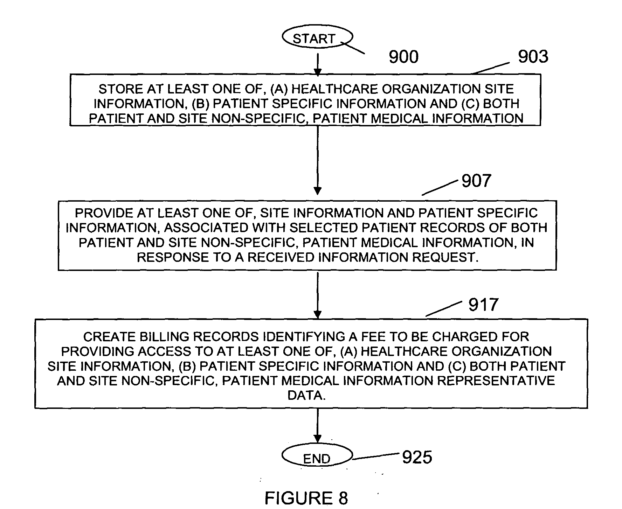

Figure 8 shows a flowchart of a process used by a service provider (SP) for providing data for a clinical trial in response to an information request. In step 903 following the start at step 900 application 20 stores in one or more repositories, at least one of, (a) healthcare organization site information, (b) patient specific information and (c) both patient and site non-specific, patient medical information. In step 907 application 20 provides at least one of, site information and patient specific information, associated with selected patient records of the patient and site non-specific, patient medical information, in response to a received information request. In another embodiment the healthcare organization site information and patient specific information data are encrypted. Application 20 in step 917 creates billing records identifying a fee to be charged for providing access to at least one of, (a) healthcare organization site information, (b) patient specific information and (c) both patient and site non-specific, patient medical information representative data. The process of Figure 8 terminates at step 925.

The systems and processes presented in Figures 1-8 are not exclusive. Other systems and processes may be derived in accordance with the principles of the invention to accomplish the same objectives. Although this invention has been described with reference to particular embodiments, it is to be understood that the embodiments and variations shown and described herein are for illustration purposes only. Modifications to the current design may be implemented by those skilled in the art, without departing from the scope of the invention. Further, any of the functions provided by the systems and process of Figures 1-8 may be implemented in hardware, software or a combination of both. The system is usable in other environments (e.g., financial, administrative, demographic) not just healthcare related environments wherever it is necessary to remove person and site specific identification information to enable the analysis and processing of aggregated data from a population of people without risking unauthorized access to confidential and sensitive person specific information. The system may be offered to users as an ASP (Application Service Provider) hosted service whereby database and analysis applications are offered with pricing models such as pay-per-trial, pay-per-patient, pay-per-data access, etc. Further, in an embodiment, the system advantageously employs multiple encryption processes associated with corresponding locations. The system advantageously enables the provision of a service supporting candidate Patient and site identification for use in identifying suitable candidate patients and trial sites for a clinical trial as documented in the figures.

## Claims

1. A system for processing medical information of a plurality of patients to provide data for use in supporting clinical decision making, comprising:
an interface for receiving, from a remote source, data representing patient non-specific medical information excluding patient specific information, said patient non-specific medical information comprising medical information of a plurality of individual patients treated at one or more healthcare organization sites; and
a data processor for automatically,
parsing said received data,
identifying, within said parsed received data, site specific information associated with a particular healthcare provider organization site and facilitating identification of said particular healthcare provider organization site,
removing said identified site specific information from said data representing patient non-specific medical information to provide both patient and site non-specific, patient medical information representative data and
storing said both patient and site non-specific, patient medical information representative data in a repository.

2. A system according to claim 1, wherein
said received patient non-specific medical information data excludes patient specific information associated with individual patients comprising at least two of, (a) a text string associated with a patient name, (b) a patient identifier, (c) patient address, (d) patient contact information, (e) patient medical insurance information, (f) a medical record number and (g) data associated with a patient healthcare provider organization or physician and including
a data extraction processor for removing said patient specific information associated with individual patients from patient medical record data to provide said patient non-specific medical information wherein
said data extraction processor removes said patient specific information by encrypting said patient specific information with a secure encryption key.

3. A system according to claim 1, including
a communication interface for communicating via a network data representing said both patient and site non-specific, patient medical information to a remote site and
an identifier generator for generating a substitute site identifier for replacing site specific information removed from said data representing patient non-specific medical information, said substitute site identifier being linked with a particular healthcare provider organization site by stored linking data and otherwise being unassociated with said particular healthcare provider organization site.

4. A system according to claim 1, wherein
said data processor stores said removed identified site specific information together with information linking removed identified site specific information with associated patient non-specific patient medical information in a database and
said database links data identifying a particular healthcare organization site of said one or more healthcare organization sites with corresponding particular patients of said plurality of individual patients.

5. A system according to claim 1, wherein
said data processor retrieves site information associated with selected patient records of said both patient and site non-specific, patient medical information in response to a received information request and
initiates communication of said retrieved site information to a requesting system.

6. A system according to claim 1, including
a query generator for generating a query for communication to a destination associated with said remote source, said query requesting access to patient specific information associated with corresponding patient non-specific medical information,
said data processor acquires requested patient specific information in response to a request for said patient specific information and
an audit processor for maintaining records identifying user access to data including to at least one of, (a) patient specific information and (b) patient non-specific medical information,
said records including at least one of, (i) identification information associated with a user attempting to access said data, (ii) identification information of data accessed and (iii) identification information of a device source of a request to access said data.

7. A system according to claim 1, wherein
said patient non-specific medical information is for use in a clinical trial and including
a billing processor for creating billing records identifying a fee to be charged for providing access to at least one of, (a) removed site specific information and (b) both patient and site non-specific, patient medical information representative data.

8. A system according to claim 1, wherein
said data processor step of removing said identified site specific information comprises encrypting said identified site specific information with a secure encryption key.

9. A system for use by a healthcare provider in processing medical information of a plurality of patients to provide data for use in supporting clinical decision making, comprising:
a data processor for automatically processing received patient medical data by,
parsing said received patient medical data,
identifying, within said parsed received patient medical data, patient specific information associated with individual patients and facilitating identification of individual patients,
removing said identified patient specific information from said parsed received patient medical data to provide patient non-specific patient medical information representative data including related healthcare provider site information and
storing said removed identified patient specific information and said patient non-specific patient medical information representative data in at least one repository; and
a communication interface for communicating at least one of, (a) said removed identified patient specific information and (b) said patient non-specific patient medical information representative data, to a remote system in response to user command.

10. A system according to claim 9, wherein
said data processor step of removing said identified patient specific information comprises encrypting said identified patient specific information and said removed identified patient specific information comprises said encrypted identified patient specific information.

11. A system according to claim 9, wherein
said communication interface communicates removed identified patient specific information associated with corresponding patient non-specific patient medical information representative data to a remote system in response to a received request for said patient specific information and
said patient specific information associated with individual patients comprises at least one of, (a) a text string associated with a patient name, (b) a patient identifier, (c) patient address, (d) patient contact information and
is used for identifying particular individual patients associated with corresponding medical data.

12. A system according to claim 9, including
an audit processor for maintaining records identifying user access to data including to at least one of, (a) patient specific information and (b) patient non-specific patient medical information representative data,
said records including at least one of, (i) identification information associated with a user attempting to access said data, (ii) identification information of data accessed and (iii) identification information of a device source of a request to access said data and including
an identifier generator for generating a substitute patient identifier for replacing patient specific information removed from said parsed received patient medical data, said substitute patient identifier being linked with a particular patient by stored linking data and otherwise being unassociated with said particular patient.

13. A system according to claim 9, including
a billing processor for creating billing records identifying a fee to be charged for providing access to at least one of, (a) removed patient specific information and (b) patient non-specific patient medical information representative data.

14. A system for processing medical information of a plurality of patients to provide data for use in supporting clinical decision making, comprising:
at least one repository including at least one of,
(a) healthcare organization site information,
(b) patient specific information and
(c) both patient and site non-specific, patient medical information,
said both patient and site non-specific, patient medical information being associated with patients identified by said patient specific information and with a healthcare organization site identified by said healthcare organization site information; and
a data processor for providing at least one of, site information and patient specific information, associated with selected patient records of said both patient and site non-specific, patient medical information, in response to a received information request.

15. A system according to claim 14, wherein
said healthcare organization site information and patient specific information data are encrypted.
